(19) Europäisches Patentamt
European Patent Office
Office européen des brevets

(11) Veröffentlichungsnummer: **0 124 013**
**B1**

(12) # EUROPÄISCHE PATENTSCHRIFT

(45) Veröffentlichungstag der Patentschrift: **13.07.88**

(21) Anmeldenummer: **84104332.6**

(22) Anmeldetag: **17.04.84**

(51) Int. Cl.⁴: **C 07 D 303/22**, C 07 D 303/04, C 07 D 303/08, C 07 D 301/02 // C07D249/08

(54) **Verfahren zur Herstellung von Oxiranen.**

(30) Priorität: **29.04.83 DE 3315524**

(43) Veröffentlichungstag der Anmeldung: **07.11.84 Patentblatt 84/45**

(45) Bekanntmachung des Hinweises auf die Patenterteilung: **13.07.88 Patentblatt 88/28**

(84) Benannte Vertragsstaaten: **BE CH DE FR GB LI**

(56) Entgegenhaltungen:
**EP-A-0 040 345**
**US-A-4 296 236**

**Annalen der Chemie, Band 611, 1958, Seiten 117-121**

(73) Patentinhaber: **BAYER AG, Konzernverwaltung RP Patentabteilung, D-5090 Leverkusen 1 Bayerwerk (DE)**

(72) Erfinder: **Zerbes, Rudolf, Dr., In der Beek 26, D-5600 Wuppertal 1 (DE)**
Erfinder: **Linke, Siegfried W., Dr. Bayer Pharma Korea Ltd., Daehan Building 10th Floor 75 Seosomondong, Choong-ku Seoul (KR)**
Erfinder: **Mohrmann, Karl Heinrich, Dr., Roonstrasse 61, D-5600 Wuppertal 1 (DE)**
Erfinder: **Reiser, Wolf, Dr., Kiebitzweg 12a, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von bekannten Oxiranen, die als Zwischenprodukte zur Synthese von Verbindungen mit pflanzenwuchsregulierender und fungizider Wirksamkeit verwendet werden können.

Es ist bereits bekannt geworden, dass man Oxirane herstellen kann, indem man Dimethylsulfoxid mit Hilfe von Methyliodid in Trimethyloxosulfoniumiodid überführt und letzteres mit Carbonyl-Verbindungen in Gegenwart einer Base umsetzt (vgl. J. Amer. Chem. Soc. 87, 1353–1364 (1965) und Liebigs Ann.Chem. 611, 117–121 (1958).

So lässt sich z.B. 2-(4-Chlorphenoxy-methyl)-2-tert.-butyl-oxiran synthetisieren, indem man aus Dimethylsulfoxid und Methyliodid zubereitetes Trimethyloxosulfoniumiodid mit 1-(4-Chlor-phenoxy)-3,3-dimethyl-butan-2-on in Gegenwart einer starken Base, wie z.B. Natriumhydrid oder Natriumamid, in Gegenwart eines inerten Verdünnungsmittels, wie z.B. Dimethylsulfoxid, umsetzt (vgl. EP-A 0 040 345). Die Ausbeuten bei diesem Verfahren sind gut. Nachteilig ist jedoch, dass zur Herstellung des als Ausgangsprodukt benötigten Trimethyloxosulfoniumiodids das relativ teure Methyliodid erforderlich ist.

Es wurde nun gefunden, dass man die bekannten Oxirane der Formel

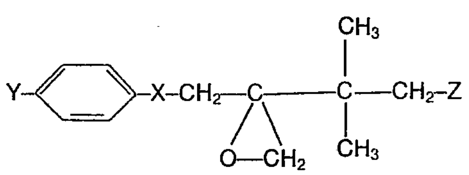
(I)

in welcher

Y für Chlor oder Phenyl steht,

X für Sauerstoff oder $CH_2$ steht und

Z für Wasserstoff oder Halogen steht,

erhält, wenn man

in einer ersten Stufe Dimethylsulfoxid mit Dimethylsulfat bei Temperaturen zwischen 60 °C und 110 °C behandelt, wobei auf 1 Mol an Dimethylsulfat 1,5 bis 4 Mol an Dimethylsulfoxid eingesetzt werden,

und in einer zweiten Stufe das dabei entstehende Trimethyloxosulfonium-methylsulfat der Formel

$$(CH_3)_3SO^{\oplus}\ CH_3SO_4^{\ominus} \qquad (II)$$

ohne vorherige Isolierung mit einem Keton der Formel

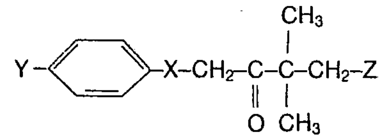
(III)

in welcher

X, Y und Z die oben angegebene Bedeutung haben,

in Gegenwart einer Base aus der Gruppe bestehend aus Natriumhydrid, Natriumamid, Alkalimetallhydroxid und Alkalimetallalkoholat, sowie in Gegenwart eines organischen Verdünnungsmittels bei Temperaturen zwischen 5 °C und 40 °C umsetzt, wobei die Molverhältnisse an Reaktionskomponenten so gewählt werden, dass auf 1 Mol an Keton der Formel (III) 1,1 bis 1,3 Mol an Trimethyloxosulfonium-methylsulfat und 1,6 bis 2,0 Mol an Base vorhanden sind.

Der Verlauf des erfindungsgemässen Verfahrens ist als äusserst überraschend zu bezeichnen. Aus dem Stand der Technik war nämlich bekannt, dass Dimethylsulfoxid mit anderen Alkylierungsreagenzien als Methyliodid in der Regel nicht am Schwefel, sondern unerwünschterweise am Sauerstoff alkyliert wird (vgl. «Sulfur Ylides in Organic Chemistry», Monography, Academic Press, 10 (1975)). Lediglich mit einem Lewis-Säure-Alkylhalogenid-Komplex läst sich Dimethylsulfoxid in schlechten Ausbeuten am Schwefel alkylieren. Es war deshalb anzunehmen, dass zur Herstellung grösserer Mengen an Trimethyloxosulfonium-Salz nur Methyliodid als Methylierungsmittel in Frage kommt. Im Gegensatz zu den Erwartungen gelingt die gewünschte S-Methylierung nach dem erfindungsgemässen Verfahren jedoch auch problemlos bei Verwendung von Dimethylsulfat.

Das erfindungsgemässe Verfahren zeichnet sich durch eine Reihe von Vorteilen aus. So ermöglicht es die Herstellung von Oxiranen der Formel (I) in guten Ausbeuten. Ausserdem sind die Ausgangsstoffe relativ preiswert und auch in technischem Massstab verfügbar. Die nach dem erfindungsgemässen Verfahren herstellbaren Oxirane sind durch die Formel (I) definiert. In dieser Formel steht X für Sauerstoff oder die $CH_2$-Gruppe, und Y steht für Chlor oder Phenyl. Der Rest Z steht vorzugsweise für Wasserstoff, Fluor oder Chlor.

Verwendet man bei dem erfindungsgemässen Verfahren neben Dimethylsulfoxid und Dimethylsulfat das 1-(4-Chlor-phenoxy)-3,3-dimethyl-butan-2-on als Ausgangsstoff und Natriummethylat als Base, so kann der Reaktionsablauf durch das folgende Formelschema veranschaulicht werden:

a) $(CH_3)_2SO + (CH_3)_2SO_4 \longrightarrow (CH_3)_3SO^{\oplus}\ CH_3SO_4^{\ominus}$

b) 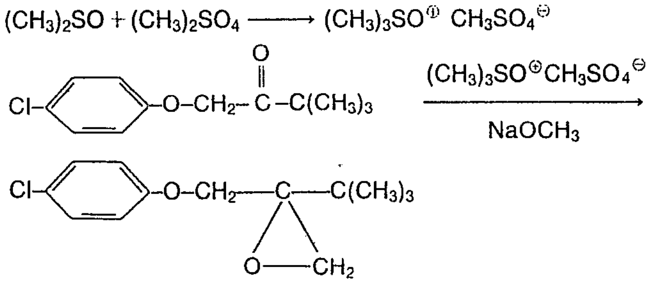

Die bei dem erfindungsgemässen Verfahren als Ausgangsstoffe benötigten Ketone sind durch die Formel (III) definiert. In dieser Formel steht Y für Chlor oder Phenyl, und X steht für Sauerstoff oder die $CH_2$-Gruppe. Der Rest Z steht vorzugsweise für Wasserstoff, Fluor oder Chlor.

Die Ketone der Formel (III) sind bekannt (vgl. DE-C 22 01 063, DE-A 27 05 678 und DE-A 27 37 489). Als Basen kommen bei der Durchführung des erfindungsgemässen Verfahrens Natriumhydrid, Natriumamid, Alkalimetallhydroxide, wie zum Beispiel Natriumhydroxid und Kaliumhydroxid, und ausserdem Alkalimetallalkoholate, wie zum Beispiel Natriummethylat, Natriumethylat und Kalium-tert.-butlat, in Betracht.

Als organische Verdünnungsmittel können bei dem erfindungsgemässen Verfahren sowohl bei der Herstellung des Trimethyloxosulfoniummethylsulfats als auch bei der anschliessenden Umsetzung dieses Stoffes mit einem Keton der Formel (III) vorzugsweise Nitrile, wie Acetonitril, ferner polare Solventien, wie Dimethylsulfoxid, und ausserdem aliphatische oder aromatische Kohlenwasserstoffe, wie Hexan, Benzol, Toluol oder Xylol, verwendet werden. – Bei der Herstellung des Trimethyloxosulfonium-methylsulfats erübrigt sich die gesonderte Zugabe eines Verdünnungsmittels, wenn man Dimethylsulfoxid in einem ausreichenden Überschuss verwendet.

Das erfindungsgemässe Verfahren wird im allgemeinen bei Normaldruck durchgeführt. Es ist aber auch möglich, bei erhöhtem oder vermindertem Druck zu arbeiten.

Im einzelnen verfährt man bei der Durchführung des erfindungsgemässen Verfahrens in der Weise, das man Dimethylsulfat und Dimethylsulfoxid gegebenenfalls in Gegenwart eines zusätzlichen Verdünnungsmittels zusammengibt, das Gemisch erhitzt, dann abkühlt und mit einer Lösung von Keton der Forrmel (III) in einem organischen Solvens versetzt und anschliessend die Base hinzufügt. Die Aufarbeitung erfolgt nach üblichen Methoden. Im allgemeinen geht man so vor, dass man das Reaktionsgemsich mit Wasser versetzt, gegebenenfalls das entstehende Gemisch mit Aktivkohle verrührt und über Kieselgur filtriert, dann die organische Phase abtrennt, wäscht und gegebenenfalls nach vorherigem Trocknen einengt. Das dabei anfallende Produkt kann zur weiteren Reinigung unter vermindertem Druck destilliert werden.

Die nach dem erfindungsgemässen Verfahren herstellbaren Oxirane der Formel (I) sind wertvolle Ausgangsstoffe zur Synthese von 1-Hydroxyethyl-azol-Derivaten, welche hervorragende pflanzenwuchsregulierende und fungizide Eigenschaften besitzen (vgl. EP-A 0 040 345).

So lässt sich beispielsweise das 2-(4-Chlorphenoxy-methyl)-3,3-dimethyl-1-(1,2,4-triazol-1-yl)-butan-2-ol der Formel

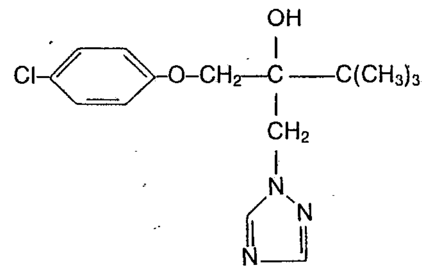

herstellen, indem man 2-(4-Chlor-phenoxy-methyl)-2-tert.-butyl-oxiran mit 1,2,4-Triazol in Gegenwart von Ethanol umsetzt. Diese Synthese lässt sich formelmässig wie folgt veranschaulichen:

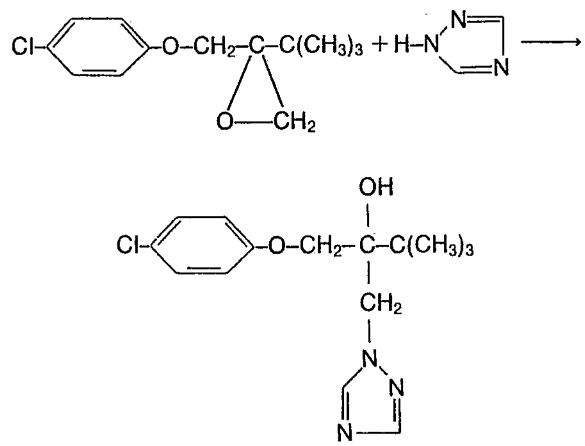

Das erfindungsgemässe Verfahren wird durch die nachfolgenden Beispiele veranschaulicht.

Beispiel 1

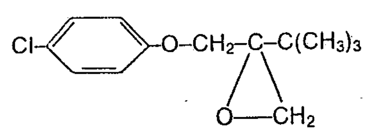

Ein Gemisch aus 71 ml (78 g; 1 Mol) Dimethylsulfoxid und 28,7 ml (37,8 g; 0,3 Mol) Dimethylsulfat wurde auf 100 °C erhitzt und 30 Minuten bei dieser Temperatur gehalten. Nach dem Abkühlen auf Raumtemperatur wurde unter Rühren eine Lösung von 57 g (0,25 Mol) 1-(4-Chlor-phenoxy)-3,3-dimethyl-butan-2-on in 100 ml Toluol zugetropft. Diese Reaktionsmischung wurde dann auf 10 °C gekühlt und unter Rühren portionsweise mit 25 g (0,46 Mol) Natriummethylat versetzt. Man rührt anschliessend weitere 12 Stunden bei Raumtemperatur und fügte dann 100 ml Wasser hinzu. Das entstehende Gemisch wurde mit 0,5 g Aktivkohle verrührt und über Kieselgur filtriert. Danach wurde die organische Phase abgetrennt, zweimal mit je 100 ml Wasser gewaschen und unter vermindertem Druck eingedampft. Es verblieb ein Rückstand von 55,3 g, der gemäss Gaschromatogramm zu 71% aus 2-(4-Chlorphenoxy-methyl)-2-tert.-butyl-oxiran bestand. Danach errechnet sich eine Ausbeute von 65,2% der Theorie.

Beispiel für die Verwendung eines erfindungsgemäss herstellbaren Oxirans zur Synthese eines 1-Hydroxyethylazol-Derivates mit pflanzenwuchsregulierender und fungizider Wirksamkeit

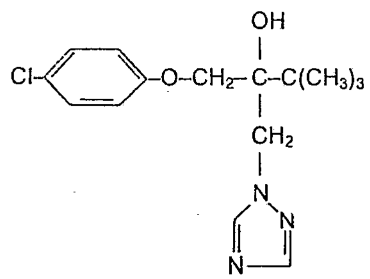

Ein Gemisch aus 72,15 g (0,3 Mol) 2-(4-Chlorphenoxy-methyl)-2-tert.-butyl-oxiran und 24,15 g (0,35 Mol) 1,2,4-Triazol in 120 ml Ethanol wurde 48 Stunden unter Rückfluss erhitzt. Anschliessend wurde eingeengt, und der verbleibende Rückstand wurde in 200 ml Essigester aufgenommen. Das entstehende Gemisch wurde zunächst kurzzeitig erhitzt und dann im Eisbad abgekühlt. Der dabei ausfallende Feststoff wurde abgesaugt und mit Essigester gewaschen. Das Filtrat wurde eingeengt, der verbleibende Rückstand wurde in einem Gemisch aus Ether/Hexan gelöst, und die entstehende Lösung wurde mit Chlorwasserstoff begast. Dabei bildete sich ein Niederschlag, der abgesaugt und mit Ether gewaschen wurde. Aus dem so hergestellten Salz wurde durch Zugabe von Essigester und 1 n wässriger Natronlauge die zugrundeliegende freie Base freigesetzt. Man erhielt auf diese Weise 60,2 g (65% der Theorie) an 2-(4-Chlor-phenoxy-methyl)-3,3-dimethyl-1-(1,2,4-triazol-1-yl)-butan-2-ol vom Schmelzpunkt 84–87 °C.

**Patentansprüche**

1. Verfahren zur Herstellung von Oxiranen der Formel

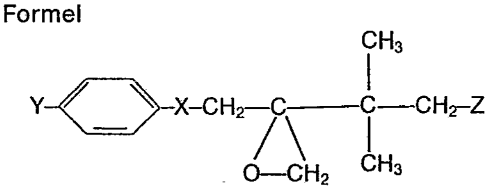 (I)

in welcher

Y für Chlor oder Phenyl steht,

X für Sauerstoff oder $CH_2$ steht und

Z für Wasserstoff oder Halogen steht,

dadurch gekennzeichnet, dass man

in einer ersten Stufe Dimethylsulfoxid mit Dimethylsulfat bei Temperaturen zwischen 60 °C und 110 °C behandelt, wobei auf 1 Mol an Dimethylsulfat 1,5 bis 4 Mol an Dimethylsulfoxid eingesetzt werden,

und in einer zweiten Stufe das dabei entstehende Trimethyloxosulfonium-methylsulfat der Formel

$$(CH_3)_3SO^{\oplus}\ CH_3SO_4^{\ominus} \quad (II)$$

ohne vorherige Isolierung mit einem Keton der Formel

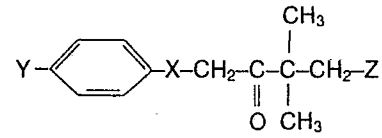 (III)

in welcher

X, Y und Z die oben angegebene Bedeutung haben,

in Gegenwart einer Base aus der Gruppe bestehend aus Natriumhydrid, Natriumamid, Alkalimetallhydroxid und Alkalimetallalkoholat, sowie in Gegenwart eines organischen Verdünnungsmittels bei Temperaturen zwischen 5 °C und 40 °C umsetzt, wobei die Molverhältnisse an Reaktionskomponenten so gewählt werden, dass auf 1 Mol an Keton der Formel (III) 1,1 bis 1,3 Mol anTrimethyloxosulfonium-methylsulfat und 1,6 bis 2,0 Mol an Base vorhanden sind.

2. Verfahren gemäss Anspruch 1, dadurch gekennzeichnet, dass man als organische Verdünnungsmittel Acetonitril, Dimethylsulfoxid oder Toluol einsetzt.

3. Verfahren gemäss Anspruch 1, dadurch gekennzeichnet, dass man als Keton der Formel (III) 1-(4-Chlorphenoxy)-3,3-dimethyl-butan-2-on einsetzt.

**Claims**

1. Process for the preparation of oxiranes of the formula

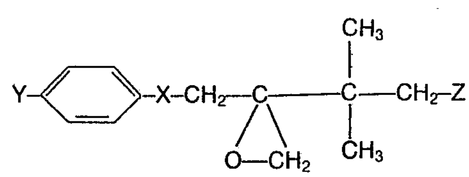 (I)

in which

Y represents chlorine or phenyl,

X represents oxygen or $CH_2$ and

Z represents hydrogen or halogen,

characterised in that, in a first step, dimethyl sulphoxide is treated with dimethyl sulphate at temperatures between 60 °C and 110 °C, 1.5 to 4 moles of dimethyl sulphoxide being employed for 1 mole of dimethyl sulphate, and in the second step, the trimethyloxosulphonium methyl sulphate, which is produced thereby, of the formula

$$(CH_3)_3SO^{\oplus}\ CH_3SO_4^{\ominus} \quad (II)$$

is reacted, without previous isolation, with a ketone of the formula

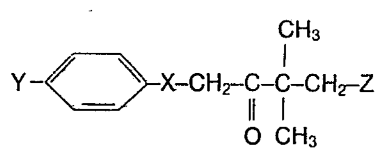 (III)

in which

X, Y and Z have the meanings indicated above, in the presence of a base from the group consisting of sodium hydride, sodium amide, alkali metal hydroxide and alkali metal alcoholate, and in the presence of an organic diluent, at temperatures between 5 °C and 40 °C, the molar ratios of the reaction components being chosen so that 1.1 to 1.3 moles of trimethyloxosulphonium methyl sulphate and 1.6 to 2.0 moles of base are present for 1 mole of ketone of the formula (III).

2. Process according to Claim 1, characterised in that acetonitrile, dimethyl sulphoxide or toluene are employed as the organic diluents.

3. Process according to Claim 1, characterised in that 1-(4-chlorophenoxy)-3,3-dimethyl-2-butanone is employed as the ketone of the formula (III).

**Revendications**

1. Procédé de production d'oxiranes de formule

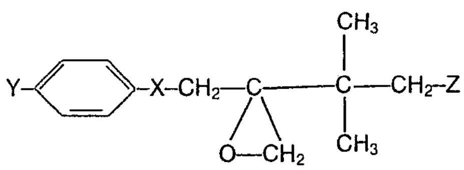

(I)

dans laquelle

Y représente le chlore ou le groupe phényle,

X représente l'oxygène ou le groupe $CH_2$ et

Z représente l'hydrogène ou un halogène,

caractérisé en ce que, dans une première étape, on traite du diméthylsulfoxyde avec du sulfate de diméthyle à des températures de 60 à 110 °C, en utilisant alors par mole de sulfate de diméthyle 1,5 à 4 moles de diméthylsulfoxyde,

et, dans une seconde étape, on fait réagir le méthylsulfate de triméthyloxosulfonium ainsi obtenu de formule

$$(CH_3)_3SO^{\oplus}\ CH_3SO_4^{\ominus} \qquad (II)$$

sans isolement préalable, avec une cétone de formule

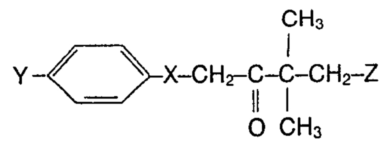

(III)

dans laquelle

X, Y et Z ont la définition indiquée ci-dessus, en présence d'une base choisie dans le groupe comprenant l'hydrure de sodium, l'amidure de sodium, un hydroxyde de métal alcalin et un alcoolate de métal alcalin, de même qu'en présence d'un diluant organique à des températures comprises entre 5 et 40 °C en choisissant alors les rapports molaires des composants réactionnels de manière qu'il y ait par mole de cétone de formule (III) 1,1 à 1,3 mole de méthylsulfate de triméthyloxosulfonium et 1,6 à 2,0 moles de base.

2. Procédé suivant la revendication 1, caractérisé en ce qu'on utilise comme diluants organiques l'acétonitrile, le diméthylsulfoxyde ou le toluène.

3. Procédé suivant la revendication 1, caractérisé en ce qu'on utilise comme cétone de formule (III) la 1-(4-chlorophénoxy)-3,3-diméthylbutane-2-one.